# EUROPEAN PATENT APPLICATION

(11) **EP 4 534 563 A1**
(43) Date of publication of application: **09.04.2025**
(21) Application number: 23811085.2
(22) Date of filing: 24.05.2023
(51) Int. Cl.: C07K 19/00, C07K 14/755, A61K 38/37, A61P 7/04

(54) **FVIII FUSION PROTEIN CONJUGATE HAVING EXTENDED HALF-LIFE AND USE THEREOF**

(30) Priority: 25.05.2022 CN 202210577314
(71) Applicant: Jiangsu Gensciences Inc., Nantong, Jiangsu 226000 (CN)
(72) Inventor: WANG, Yali, Nantong, Jiangsu 226000 (CN); GAO, Jie, Nantong, Jiangsu 226000 (CN); CHEN, Xian, Beijing 100176 (CN); MO, Weichuan, Beijing 100176 (CN); SU, Hongsheng, Beijing 100176 (CN)
(74) Representative: Studio Torta S.p.A.
(86) International application number: PCT/CN2023/095973
(87) International publication number: WO 2023/227015

(57) **Abstract**

Provided in the present disclosure are a blood coagulation factor VII-Fc fusion protein conjugated to polyethylene glycol and having an extended cyclic half-life, a preparation method therefor, and the use thereof.

## Description

This application claims the priority of Chinese Patent Application No. 202210577314.2, filed on May 25, 2022, and the disclosure of which is hereby incorporated by reference.

### FIELD

The present invention relates to the field of biomedicine, and in particular to a recombinant human coagulation factor VIII fusion protein conjugate with prolonged half-life and a preparation method and uses thereof.

### BACKGROUND

Hemophilia A is a hereditary bleeding disorder caused by the insufficient activity or dysfunction of coagulation factor VIII (FVIII), and for which, supplement of active FVIII is an effective therapy treatment. FVIII gene is one of the longest genes cloned so far, and FVIII molecule is a protein drug with the largest molecular weight used in clinical practice. Due to the short half-life of FVIII in the blood, which is only 8-12 hours, patients with severe hemophilia A who need to be given preventive treatment have to be injected with FVIII intravenously (i.v.) about 3 times a week.

A monomer-dimer hybrid recombinant FVIII-Fc fusion protein (Eloctate) developed by Bioverativ (US) was approved for marketing by the US FDA in June 2014. Clinical data show that its half-life in the human body is 18.8 h, which is only 1.5-1.7 times longer than that of natural protein (Dumont JA et al., Blood, 2012, 119: 3024-3030; Powell JS et al., Blood, 2012, 119: 3031-3037), and injection is needed every 3 to 5 days. However, in HEK-293 cells transfected with dual expression vector of rFVIII Fc and Fc constructed by Bioverativ, rFVIII-Fc homodimers fusion protein was not detected in the expression products as expected, and only monomer-dimer hybrid rFVIII-Fc fusion protein and Fc dimer were expressed.

For the preparation of long-acting formulations of protein drugs, polymers with high solubility (such as polyethylene glycol (PEG)) are commonly used for chemical modification of the surface of protein drugs. Generally, the higher the modification rate, the more obvious the reduction of protein antigenicity and activity. Examples of using polyethylene glycol (PEG) to extend the half-life of FVIII have been reported. Novonordisk (N8-GP), Bayer (BAY94-9027) and Baxter (Bax 855) have all developed PEGylated long-acting FVIII products which have entered clinical research. However, pharmacokinetic research data showed that PEGylated FVIII did not show a significantly prolonged half-life (Tiede A et al., J Thromb Haemost. 2013; 11: 670-678); (Coyle T et al., Haemophilia. 2012; 18( Suppl 3): 22); (Turecek PL et al., Hamostaseologie, 2012, 32 Suppl 1: S29-38).

Patients with hemophilia need a life-long transfusion of coagulation factors to stop bleeding and prevent bleeding. Therefore, it is necessary to develop coagulation factors with a longer half-life to reduce administration frequency. In addition, how to maintain good biological activity while extending the half-life of the proteins is also a difficult problem to be solved.

### SUMMARY

WO2019219049A1 discloses a PEG-modified FVIII-Fc fusion protein, wherein the PEGylated fusion protein can reduce the severe bleeding rate and 12h rebleeding rate in hemophilia A mouse model (tail vein transection model), and increase the 48h survival rate of model animals.

In further research, the inventor unexpectedly found that not any Fc region and any number of PEG modifications have a similar effect on prolonging the half-life of the FVIII-Fc fusion protein. For a specific FVIII fragment, the Fc region derived from IgG and a particular number of PEG modification can bring unexpectedly significantly extended half-life. Clinical data show that the PEGylated fusion protein of the present invention has a half-life of 30.87 hours to 31.91 hours, which is 2.10-2.25 times higher than that of Advate and 2.61-2.80 times higher than that of the natural protein, so that it can be administered at a frequency of once a week, greatly reducing the frequency of drug injection. Therefore, patients who need long-term medication can work and study normally on weekdays, and only need to be administered on weekends, which greatly improves patient compliance and significantly improves the life quality of patients. Based on the above, the present invention has been accomplished.

The present invention provides the following technical solutions:
The first aspect of the present invention provides a conjugate of a coagulation factor VIII-Fc fusion protein and polyethylene glycol (PEG), wherein the coagulation factor VIII-Fc fusion protein comprises an active moiety of the coagulation factor VIII (FVIII) and an Fc fragment, the active moiety of the coagulation factor VIII (FVIII) is directly connected to the Fc fragment or indirectly linked to the Fc fragment through a linker to form the fusion protein, the conjugate is modified with PEG at an average PEG number of 3-8, 3-7, 3-6, 4-8, 4-7, 4-6 or 4.2-5.1, preferably 4.2-5.1. The average PEG number of modification is the molar ratio of the PEG to the fusion protein of the conjugate; or it can also be understood as the average number of PEGs carried by each modified fusion protein in the sample, which contains more than one fusion protein. Due to the different number of PEGs carried by each modified fusion protein, its average value may be either an integer or a non-integer.

In some embodiments, a modifier for PEG modification has a structure represented by formula (2): 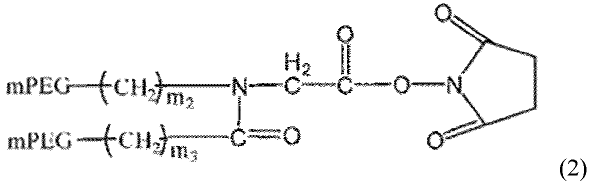
wherein, 0≤m2≤6, preferably, m2 is 2; 0≤m3≤6, preferably, m3 is 1; mPEG- represents a methoxy mono-capped polyethylene glycol group; and
preferably, the PEG has a molecular weight of 30 kD-50 kD, preferably 40 kD.

In some embodiments, the active moiety of the coagulation factor VIII is full-length or truncated human coagulation factor VIII, preferably B-domain truncated human coagulation factor VIII, more preferably a polypeptide comprising an amino acid sequence set forth in SEQ ID NO: 1 or SEQ ID NO: 2, or a polypeptide having at least 90%, 95% or higher identity to the amino acid sequence set forth in SEQ ID NO: 1 or SEQ ID NO: 2 and still having FVIII activity.

In other embodiments, the Fc fragment is an Fc fragment derived from IgG, preferably IgG1, IgG2 or IgG4, more preferably IgG2.

In other embodiments, the Fc fragment comprises an amino acid sequence selected from the group consisting of:
(i) the amino acid sequence set forth in SEQ ID NO: 3;
(ii) the amino acid sequence set forth in SEQ ID NO: 4; and
(iii) the amino acid sequence set forth in SEQ ID NO:5.

In other embodiments, the conjugation of the fusion protein and PEG is at a random or specific position, and the conjugation position is selected from the group consisting of a free amino group, sulfhydryl group, sugar group and carboxyl group, preferably a free amino group.

In other embodiments, the linker includes a flexible unit and a rigid unit.

In other embodiments, the flexible unit comprises 2 or more amino acid residues selected from the group consisting of glycine, serine, alanine and threonine, preferably, the flexible unit has a general formula of (GS)ₐ(GGS)_{b}(GGGS)_{c}(GGGGS)_{d}, wherein a, b, c and d are integers greater than or equal to 0, and a+b+c+d≥1.

Preferably, the flexible unit comprises an amino acid sequence selected from the group consisting of:
(i) GSGGGSGGGGSGGGGS (SEQ ID NO: 6),
(ii) GSGGGGSGGGGSGGGGSGGGGSGGGGS (SEQ ID NO: 7),
(iii) GGGGSGGGGSGGGGSGGGGS (SEQ ID NO: 8),
(iv) GSGGGGSGGGGSGGGGSGGGGSGGGGSGGGGSGGGGS (SEQ ID NO: 9), and
(v) GGGSGGGSGGGSGGGSGGGS (SEQ ID NO: 10).

In other embodiments, the rigid unit is the carboxy terminal peptide of human chorionic gonadotropin β subunit, or the rigid unit has at least 70%, 80%, 90%, 95% or higher identity to the amino acid sequence of the carboxy terminal peptide of human chorionic gonadotropin β subunit; the rigid unit comprises 1, 2 or more glycosylation sites.

Preferably, the rigid unit comprises an amino acid sequence selected from the group consisting of:
(i) PRFQDSSSSKAPPPPSLPSPSRLPGPSDTPILPQ (SEQ ID NO: 11),
(ii) SSSSKAPPPSLPSPSRLPGPSDTPILPQ (SEQ ID NO: 12),
(iii) SSSSKAPPPS (SEQ ID NO: 13),
(iv) SRLPGPSDTPILPQ (SEQ ID NO: 14), and
(v)GSGGGGSGGGGSGGGGSGGGGSGGGSSSSSKAPPPPSLPSPSRLPGPSDTPILP Q (SEQ ID NO: 15).

In other embodiments, the coagulation factor VIII-Fc fusion protein sequentially comprises, from the nitrogen terminal to the carbon terminal, B-domain truncated human coagulation factor VIII, a flexible unit, a rigid unit and an Fc fragment, wherein:
the B-domain truncated human coagulation factor VIII comprises an amino acid sequence set forth in SEQ ID NO: 2, the flexible unit comprises an amino acid sequence set forth in SEQ ID NO: 7, the rigid unit comprises an amino acid sequence set forth in SEQ ID NO: 12, the Fc fragment comprises an amino acid sequence set forth in SEQ ID NO: 4;
the B-domain truncated human coagulation factor VIII comprises an amino acid sequence set forth in SEQ ID NO: 2, the flexible unit comprises an amino acid sequence set forth in SEQ ID NO: 6, the rigid unit comprises an amino acid sequence set forth in SEQ ID NO: 11, the Fc fragment comprises an amino acid sequence set forth in SEQ ID NO: 4;
the B-domain truncated human coagulation factor VIII comprises an amino acid sequence set forth in SEQ ID NO: 2, the flexible unit comprises an amino acid sequence set forth in SEQ ID NO: 7, the rigid unit comprises an amino acid sequence set forth in SEQ ID NO: 12, the Fc fragment comprises an amino acid sequence set forth in SEQ ID NO: 5;
   or
the B-domain truncated human coagulation factor VIII comprises an amino acid sequence set forth in SEQ ID NO: 2, the flexible unit comprises an amino acid sequence set forth in SEQ ID NO: 6, the rigid unit comprises an amino acid sequence set forth in SEQ ID NO: 11, the Fc fragment comprises an amino acid sequence set forth in SEQ ID NO: 5.

In some embodiments, the PEG molecule is linked to a primary amine (-NH₂) group on a lysine residue in the coagulation factor VIII-Fc fusion protein through an active group succinimidyl carboxymethyl ester (SCM);
preferably, the modifier for PEG modification has a structure represented by formula (2): 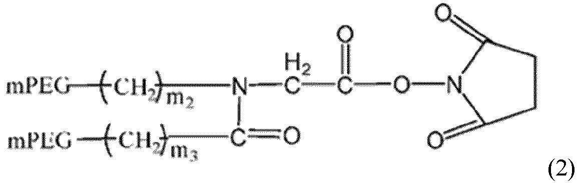
wherein, m2 is 2; m3 is 1; the molecular weight of the PEG is 40 kD.

The second aspect of the present invention provides a pharmaceutical composition comprising the aforementioned conjugate and a pharmaceutically acceptable carrier.

The third aspect of the present invention provides use of the conjugate in the manufacture of a medicament for preventing and/or treating hemorrhagic diseases. Preferably, the hemorrhagic disease is selected from the group consisting of hemorrhagic diseases in patients with congenital or acquired deficiency of FVIII, and spontaneous or surgical bleeding in patients with hemophilia A.

The fourth aspect of the present invention provides a method for preventing and/or treating hemorrhagic diseases, comprising administering the conjugate to a subject in need thereof, preferably, the hemorrhagic disease is selected from the group consisting of hemorrhagic diseases in patients with congenital or acquired deficiency of FVIII, and spontaneous or surgical bleeding in patients with hemophilia A.

The present invention further provides a method for preparing the conjugate described in the first aspect, comprising:
1) preparing the coagulation factor VIII-Fc fusion protein;
2) reacting the fusion protein obtained in step 1) with PEG, wherein the PEG and the fusion protein are in a molar ratio of (50-120): 1, preferably 100: 1, and the PEG has a molecular weight of 30-50 kD, and is preferably a branched PEG of 40 kD; and
3) purifying the conjugate obtained in step 2).

In some embodiments, the reaction is carried out at 20°C±5°C for 1-3 hours, preferably 2 hours.

### BRIEF DESCRIPTION OF DRAWINGS

In order to more clearly illustrate the embodiments of the present invention and the technical solutions of the prior art, the following briefly introduces the drawings used in the embodiments and the prior art. Apparently, the drawings in the following description are only some examples of the present invention, and those skilled in the art can obtain other embodiments according to these drawings without making creative efforts.
FIG. 1 shows the purity spectrum of FL1G2-40Y in SEC-HPLC.
FIG. 2 shows the RI chromatogram of hFVIII fusion protein at a concentration of 0.20 mg/mL.
FIG. 3 shows the RI chromatogram of PEG standard solution at a concentration of 0.20 mg/mL.
FIG. 4 shows the UV chromatogram of hFVIII fusion protein at a concentration of 0.20 mg/mL.
FIG. 5 shows the RI chromatogram of the purified conjugate at a concentration of 0.20 mg/mL.
FIG. 6 shows the UV chromatogram of the purified conjugate at a concentration of 0.20 mg/mL.

### DETAILED DESCRIPTION

In order to make the objectives, technical solutions, and advantages of the present disclosure clearer, the following further describes the present disclosure in detail with reference to the drawings and embodiments. Apparently, the described embodiments are only a part of the embodiments of the present disclosure, rather than all the embodiments. Based on the embodiments of the present disclosure, all other embodiments obtained by those of ordinary skill in the art without making creative work shall fall within the protection scope of the present disclosure.

The term "coagulation factor VIII", also called factor VIII, or FVIII, refers to a large and complex glycoprotein mainly produced by liver cells. The term "coagulation factor VIII active moiety" refers to a moiety of the fusion protein of the present disclosure that exhibits FVIII activity. Natural human FVIII consists of 2351 amino acids, including signal peptide, as well as several different domains defined by homology: three A domains, one B domain and two C domains in the order of NH₂-A1-A2-B-A3-C1-C2-COOH. In the blood, FVIII is secreted as a heterodimer consisting of two chains (cleaved at the boundary of B-A3), which are linked by divalent metal ions. The A1-A2-B chain is called the heavy chain (HC), and the A3-C1-C2 chain is called the light chain (LC).

The endogenous factor VIII molecules circulate in the body as a pool of molecules with B domains of different sizes. The gradual enzymatic cleavage of B domain may occur *in vivo,* resulting in a pool of molecular with B domains of different sizes. It is generally believed that the occurrence of the cleavage at position 740 (the last part of the B domain is excised here) is related to the activation of thrombin.

The "coagulation factor VIII" in the present disclosure may refer to the natural wild-type sequence (such as SEQ ID NO: 1), and also its variants, for example, a variant protein obtained by substitutions, deletions or insertions of one or more amino acids, while retaining the activity of coagulation factor VIII.

In an embodiment, coagulation factor VIII is a B-domain truncated molecule, wherein the remaining domains substantially correspond to amino acids 1-745 and 1640-2332 in SEQ ID NO: 1. In addition, the B-domain truncated molecule of the present disclosure may have slight differences from the sequence set forth in SEQ ID NO: 2, i.e., the remaining domains (i.e., three A domains and two C domains) may have substitutions, additions or deletions of one or more amino acids based on the amino acid sequence set forth in SEQ ID NO: 2, for example have 1, 2, 3, 4, 5, 6, 7, 8, 9, 10 or more amino acid differences or about 1 %, 2%, 3%, 4% or 5% difference from the sequence set forth in SEQ ID NO: 2. Such differences may change the binding ability of factor VIII to various other components (such as LRP, multiple receptors, other coagulation factors, cell surface), or introduce and/or eliminate glycosyl groups, while retaining the basic activity of factor VIII.

The Fc domain can also be modified to change its functions, such as complement binding and/or binding to certain Fc receptors. Mutations at positions 234, 235, and 237 in the IgG Fc domain usually result in reduced binding to FcyRI receptors, and may also lead to reduced binding to FcyRIIa and FcyRIII receptors. Such mutations do not change the binding to the FcRn receptor, which promote a long circulation half-life through the endocytosis and recirculation pathway. Preferably, the modified IgG Fc domain of the fusion protein in the present disclosure contains one or more of such mutations, some mutations (L234A, L235E, and G237A) lead to decreased affinity for certain Fc receptors, and others (A330S and P331S) lead to decreased C1q-mediated complement binding.

The polyethylene glycol (PEG) in the present invention may be linear or branched. The backbone of a branched polymer is well-known in the art. Generally, the branched polymer has a central branched core portion and one or more linear polymer chains connected to the central branched core. The present disclosure preferably uses branched form of PEG. In an embodiment, the branched polyethylene glycol can be represented by the general formula R(-PEG-OH)ₘ, where R represents the core part, such as glycerol or pentaerythritol, and m represents the number of arms.

In an embodiment, the number of branches in a branched PEG (such as mPEG, also named methoxy-PEG) is 2, so it is also called "Y-type" PEG (such as mPEG), that is, a branched PEG containing two PEGs or a branched PEG containing linear methoxy PEG.

In an embodiment of the present invention, PEG modification (i.e., conjugation), and more preferably mPEG modification is used, wherein the modification is at a random or specific position, and the modification position is selected from the group consisting of a free amino group, sulfhydryl group, sugar group and/or carboxyl group, preferably a free amino group.

In the present invention , a PEG molecule used for cross-linking reactions with protein(s) are also referred to as modifier. It is usually activated polyethylene glycol (also known as polyethylene glycol modifier or PEG modifier), that is, polyethylene glycol with functional groups (such as active linker). The terms "modifier(s)", "modifier(s) for PEG modification", "PEG modifier(s)" and "polyethylene glycol modifier(s)" can be used interchangeably herein.

In a specific embodiment of the present invention, the modifier used for mPEG random modification at free amino group can be selected from the group consisting of mPEG-SS (methoxy polyethylene glycol-succinimidyl succinate), mPEG-SC (methoxy polyethylene glycol-succinimidyl carbonate), mPEG-SPA (methoxy polyethylene glycol-succinimidyl propionate), mPEG-SG (methoxy polyethylene glycol-succinimidyl glutarate) and so on. The modifier used for N-terminal modification is selected from the group consisting of mPEG-ALD (methoxy polyethylene glycol-acetaldehyde), mPEG-pALD (methoxy polyethylene glycol-propionaldehyde), mPEG-bALD (methoxy polyethylene glycol-butyraldehyde) and so on. The modifiers mPEG-SS, mPEG-SC, mPEG-SPA, mPEG-SG, mPEG-ALD, mPEG-pALD, mPEG-bALD are linear or branched.

In a specific embodiment of the present invention, the modifier used for random modification at free sulfhydryl group is selected from the group consisting of mPEG-mal (methoxy polyethylene glycol-maleimide), mPEG-OPSS (methoxy polyethylene glycol-orthopyridyl disulfide), mPEG-Vinylsulfone (methoxypolyethylene glycol-vinylsulfone), mPEG-Thiol (methoxypolyethylene glycol-thiol), etc.

In a specific embodiment of the present invention, the modifier used for random modification at the sugar group and/or carboxyl group is mPEG-ZH (methoxy polyethylene glycol-hydrazide).

In an embodiment of the present invention, the modifier used for mPEG modification has a structure represented by formula (1): 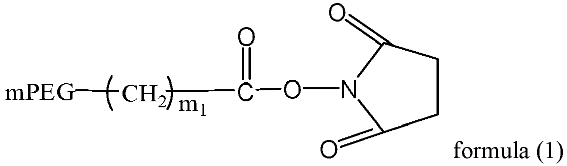 wherein, 0≤m1≤6, and m1 is preferably 5; and mPEG represents a monomethoxy-capped polyethylene glycol group. The modifier represented by formula (1) has a molecular weight of 5 kD-60 kD (kD, kilodaltons), preferably 40 kD. Preferably, in an embodiment of the present disclosure, the modifier represented by formula (1) is used for a random mPEG modification at free amino group.

In an embodiment of the present invention, the modifier used for mPEG modification has a structure represented by formula (2): 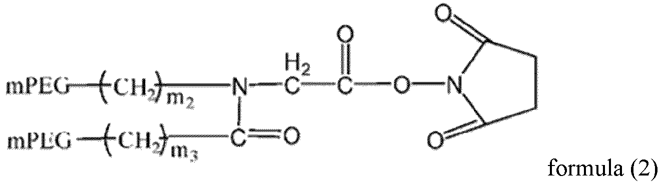 wherein, 0≤m2≤6, and m2 is preferably 2; 0≤m3≤6, and m3 is preferably 1; and mPEG represents a monomethoxy-capped polyethylene glycol group. The modifier represented by formula (2) has a molecular weight of 5 kD-60 kD, preferably 40 kD. Preferably, in an embodiment of the present disclosure, the modifier represented by formula (2) is used for a random mPEG modification at free amino group.

The size of the polymer backbone may vary, but the typical size range of polymers (such as PEG, mPEG, PPG, or mPPG) is about 0.5 kD to about 160 kD, for example, about 1 kD to about 100 kD. More specifically, the size of each hydrophilic polymer conjugated in the present disclosure mainly varies within the following ranges: about 1 kD to about 80 kD, about 2 kD to about 70 kD; about 5 kD to about 70 kD; about 10 kD to about 60 kD, about 20 kD to about 50 kD; about 30 kD to about 50 kD or about 30 kD to about 40 kD. It should be understood that these sizes represent approximate values, not precise measurements, as recognized in the art.

In a specific embodiment, the size of PEG or mPEG used in the present disclosure is above 35 kD (i.e. not less than 35 kD), preferably not less than 40 kD, not less than 45 kD, not less than 50 kD, not less than 55 kD, not less than 60 kD, not less than 65 kD or not less than 70 kD, for example, the molecular weight is specifically 40 kD, 50 kD, 60 kD, 70 kD, 80 kD, 90 kD, 100 kD, 110 kD, 120 kD, 130 kD, 140 kD, 150 kD or 160 kDa.

In a specific embodiment, the coagulation factor VIII-Fc fusion protein sequentially comprises, from the nitrogen terminal to the carbon terminal, B-domain truncated human coagulation factor VIII, a flexible unit, a rigid unit and an Fc fragment, wherein:
the B-domain truncated human coagulation factor VIII comprises an amino acid sequence set forth in SEQ ID NO: 2, the flexible unit comprises an amino acid sequence set forth in SEQ ID NO: 7, the rigid unit comprises an amino acid sequence set forth in SEQ ID NO: 12, the Fc fragment comprises an amino acid sequence set forth in SEQ ID NO: 4 (FL1G2-0);
the B-domain truncated human coagulation factor VIII comprises an amino acid sequence set forth in SEQ ID NO: 2, the flexible unit comprises an amino acid sequence set forth in SEQ ID NO: 6, the rigid unit comprises an amino acid sequence set forth in SEQ ID NO: 11, the Fc fragment comprises an amino acid sequence set forth in SEQ ID NO: 4 (FL2G2-0);
the B-domain truncated human coagulation factor VIII comprises an amino acid sequence set forth in SEQ ID NO: 2, the flexible unit comprises an amino acid sequence set forth in SEQ ID NO: 7, the rigid unit comprises an amino acid sequence set forth in SEQ ID NO: 12, the Fc fragment comprises an amino acid sequence set forth in SEQ ID NO: 5 (FL1G4-0);
   or
the B-domain truncated human coagulation factor VIII comprises an amino acid sequence set forth in SEQ ID NO: 2, the flexible unit comprises an amino acid sequence set forth in SEQ ID NO: 6, the rigid unit comprises an amino acid sequence set forth in SEQ ID NO: 11, the Fc fragment comprises an amino acid sequence set forth in SEQ ID NO: 5 (FL2G4-0).

In another specific embodiment, the conjugate is FL1G2-0, FL2G2-0, FL1G4-0 or FL2G4-0 randomly modified with PEG.

In another specific embodiment, the random modification comprises linking the PEG molecule to a primary amine (-NH₂) group on a lysine residue in the fusion protein via an active group (linker) succinimidyl carboxymethyl ester (SCM).

In another specific embodiment, the PEG is a branched-chain PEG (Y-type PEG) comprising two PEGs or linear methoxy PEGs.

In another specific embodiment, the PEG has a structure represented by formula (2), preferably, in the formula (2), m2 is 2; m3 is 1; and the molecular weight of the PEG is 40 kD.

In another specific embodiment, the PEG modifier represented by formula (2) connects PEG molecules to fusion protein molecules by forming amide bonds (as shown in formula (4)) with primary amines on lysine residues.

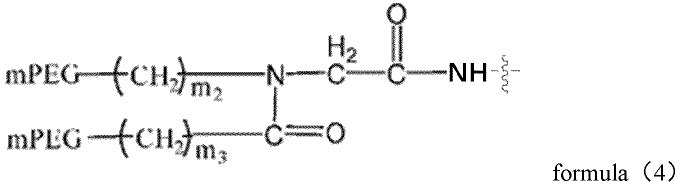

In another specific embodiment, the PEG in the conjugate has a structure represented by formula (5):

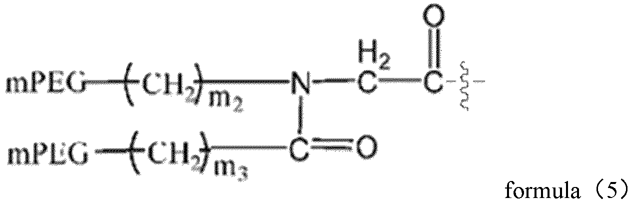

The term "improved circulation half-life" means that the molecules of the present disclosure have an altered circulation half-life, preferably an increased circulation half-life compared with wild-type factor VIII. The circulation half-life is preferably increased by at least 10%, preferably at least 15%, preferably at least 20%, preferably at least 25%, preferably at least 30%, preferably at least 35%, preferably at least 40%, preferably at least 45%, preferably at least 50%, preferably at least 55%, preferably at least 60%, preferably at least 65%, preferably at least 70%, preferably at least 75%, preferably at least 80%, preferably at least 85%, preferably at least 90%, preferably at least 95%, preferably at least 100%, more preferably at least 125%, more preferably at least 150%, more preferably at least 175%, more preferably at least 200%, and most preferably at least 250% or 300%. Even more preferably, the molecule has a circulation half-life increased by at least 400%, 500%, 600%, or even 700%.

The term "pharmaceutically acceptable carrier" includes, but is not limited to: saline, buffer, glucose, water, glycerol, ethanol, and combinations thereof. Generally, the pharmaceutical preparation should be suitable for the mode of administration. The pharmaceutical composition of the present disclosure can be made into an injection form, for example, by conventional methods with physiological saline or an aqueous solution containing glucose and other adjuvants. The pharmaceutical composition should be manufactured under aseptic conditions. The amount of active ingredient administered is a therapeutically effective amount. The pharmaceutical preparation of the invention can also be made into a sustained-release preparation.

### Examples

### Example 1 Preparation and purification of hFVIII fusion proteins

A series of hFVIII fusion protein expression plasmids were constructed according to the molecular cloning technology well known to those skilled in the art, and the expression plasmids were respectively transfected into DHFR-deficient CHO cells (see U.S. Patent 4,818,679) to express each hFVIII fusion protein listed in Table 1. For the preparation and purification steps of the fusion proteins, see Chinese Patent ZL201610692838.0 and publication WO2019219049A1, which are incorporated herein by reference in their entirety.

**Table 1 Compositions and sequences of fusion proteins**

| Name and abbreviation of hFVIII fusion protein | hFVIII moiety | Fc moiety | Linker |
|---|---|---|---|
| hFVIII-L1-G1(FL1G1-0) | B-domain truncated hFVIII (SEQ ID NO: 2) | Fc of IgG1 SEQ ID NO: 3 | SEQ ID NO: 7-SEQ ID NO: 12* |
| hFVIII-L2-G1(FL2G1-0) | B-domain truncated hFVIII (SEQ ID NO: 2) | Fc of IgG1 SEQ ID NO: 3 | SEQ ID NO: 6-SEQ ID NO: 11** |
| hFVIII-L1-G2(FL1G2-0) | B-domain truncated hFVIII (SEQ ID NO: 2) | Fc of IgG2 SEQ ID NO: 4 | SEQ ID NO: 7-SEQ ID NO: 12 |
| hFVIII-L2-G2(FL2G2-0) | B-domain truncated hFVIII (SEQ ID NO: 2) | Fc of IgG2 SEQ ID NO: 4 | SEQ ID NO: 6-SEQ ID NO: 11 |
| hFVIII-L1-G4(FL1G4-0) | B-domain truncated hFVIII (SEQ ID NO: 2) | Fc of IgG4 SEQ ID NO: 5 | SEQ ID NO: 7-SEQ ID NO: 12 |
| hFVIII-L2-G4(FL2G4-0) | B-domain truncated hFVIII (SEQ ID NO: 2) | Fc of IgG4 SEQ ID NO: 5 | SEQ ID NO: 6-SEQ ID NO: 11 |

| | | | |
|---|---|---|---|
| Note: B-domain truncated hFVIII is abbreviated as BDD FVIII, which consists of a 90 kD A1-A2 heavy chain and a 80 kD light chain. *SEQ ID NO: 7-SEQ ID NO: 12 means that the linker is formed by connecting the rigid unit set forth in SEQ ID NO: 12 to the C-terminus of the flexible unit set forth in SEQ ID NO: 7; and **SEQ ID NO: 6-SEQ ID NO: 11 means that the linker is formed by connecting the rigid unit set forth in SEQ ID NO: 11 to the C-terminus of the flexible unit set forth in SEQ ID NO: 6. hFVIII-L1-G1 and hFVIII-L2-G1 fusion proteins have been disclosed in WO2019219049A1, corresponding to hFVIII-L1-Fc(FL1F-0) and hFVIII-L2-Fc(FL2F-0) respectively, which are used as control in this application. | | | |

### Example 2 Preparation and purification of mPEG-modified hFVIII fusion protein

40 kD PEG-modified FL1G1-40Y (40 kD, Y-type PEG-modified FL1G1-0, and the subsequent naming rules are the same) was prepared and purified as described in WO2019219049A1.

In this example, 40 kD PEG-modified hFVIII fusion protein FL1G2-40Y was prepared and purified according to the following method.

Y-type Y-SCM-40K PEG was subjected to cross-linking reaction with hFVIII fusion protein. Y-SCM-40K PEG has a molecular weight of 40 kD and an active group (linker) SCM (having a molecular formula as shown in formula (2), where m2 is 2 and m3 is 1, purchased from Beijing Jenkem Technology Co., Ltd., Y-NHS-40K). The active linker of PEG was a succinimidyl carboxymethyl ester, which is easy to react with a primary amine (-NH₂) group on a lysine residue in a protein to form a stable amide bond, thereby obtaining a protein-PEG cross-linked product. Y-SCM-40K PEG and hFVIII fusion protein FL1G2-0 after filtration and concentration were weighed respectively and used for preparingo conjugates according to different molar ratios of PEG and fusion protein, such as 30:1, 50:1, 100:1, and 120:1. The conjugate prepared according to the molar ratio of 100:1 (the conjugate of this example) showed excellent prolonged half-life in the subsequent tests. The conjugate was prepared as follows: the materials were mixed according to a molar ratio of PEG: protein of 100:1 (a mass ratio of 10.26:1) for 2 h cross-linking reaction at 20°C±5°C. After cross-linking, the product was filtered through a 0.2 µm filter membrane, and then temporarily stored at 2-8°C for later purification.

Purification: First, separation was performed using S200 (GE healthcare) molecular sieve chromatograph. The chromatography column was equilibrated using a binding buffer (20 mM His-HCl, 0.1 M NaCl, 5 mM CaCl₂, 0.02% Tween 80, pH 6.8-7.2) at a linear flow rate of 150 cm/h for 3-5 column volumes (CV). The sample was loaded at a linear flow rate of 150 cm/h. After the loading of sample was completed, the chromatography column was washed using an equilibrating buffer (20 mM His-HCl, 200 mM NaCl, 5 mM CaCl₂, 0.02% Tween 80, pH 6.8-7.2) at a linear flow rate of 150 cm/h for 3-5 column volumes (CV), and equilibrated to the same pH and conductivity as the buffer. Elution was performed using a buffer (20 mM His-HCl, 0.1 M NaCl, 5 mM CaCl₂, 0.02% Tween 80, pH 6.8-7.2). Peaks with A280/260 greater than 1.8 were collected. In the second step, separation was performed using Source 15Q (GE healthcare) anion chromatography column. The chromatography column was equilibrated using a binding buffer (20 mM His-HCl, 0.1M NaCl, 5 mM CaCl₂, 0.02% Tween 80, pH 6.8-7.2) at a linear flow rate of 150 cm/h for 3-5 column volumes (CV). The sample obtained after the first step of molecular sieve chromatography was loaded at a linear flow rate of 150 cm/h. After the loading of sample was completed, the chromatography column was washed using an equilibrating buffer (20 mM His-HCl, 0.1M NaCl, 5 mM CaCl₂, 0.02% Tween 80, pH 6.8-7.2) at a linear flow rate of 150 cm/h for 3-5 column volumes (CV), and equilibrated to the same pH and conductivity as the buffer. Elution was performed using an eluting buffer (20 mM His-HCl, 2 M NaCl, 5 mM CaCl₂, 0.02% Tween 80, pH 6.8-7.2) according to 0-100% at a linear flow rate of 100 cm/h. The elution peaks with A280/260 greater than 1.8 were collected in separate tubes. The determination of purity and average PEG modification number were conducted respectively.

### Determination of purity of FL1G2-40Y by SEC-HPLC

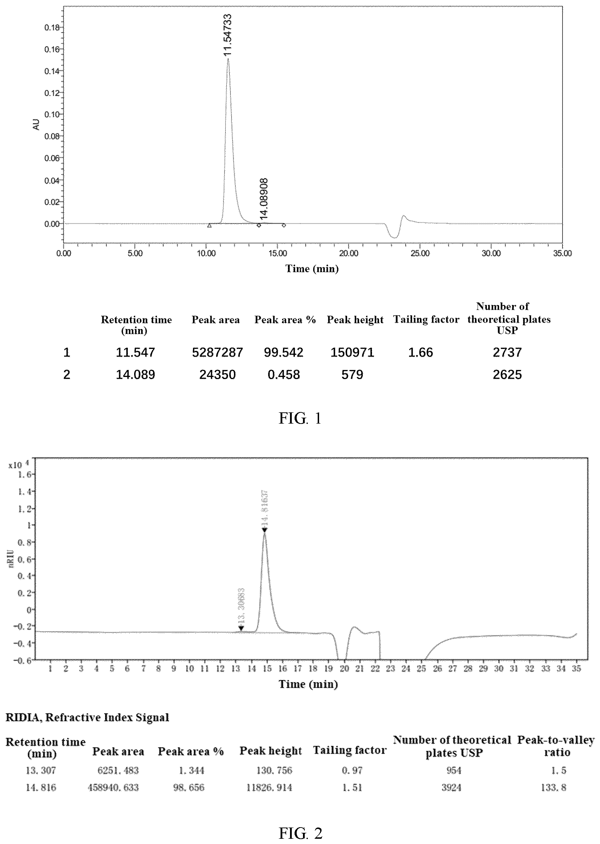

Determination was carried out using TSKgel UltraSW Aggregate chromatography column (7.8 mm×300 mm, 3 µm), and a mobile phase (0.3 mol/L arginine, 0.2 mol/L sodium chloride, 0.01 mol/L anhydrous calcium chloride, 0.02 mol/L histidine, 0.02% poloxamer 188, pH 7.0)-10% acetonitrile. Elution was carried out isocratically at a flow rate of 0.5 mL/min and a UV detection wavelength of 280 nm. The sample to be tested was diluted with a dilution buffer to a concentration of about 0.30 mg/mL. 100 µL of the test solution was injected into the liquid chromatograph, and the purity of the test substance was calculated according to the area normalization method. As shown in FIG. 1, the purity of the sample to be tested was 99.5%.

Determination of average number of PEG modification of FL1G2-40Y by combined method of SEC- HPLC- UV-RID

Determination was carried out using TSKgel UltraSW Aggregate chromatography column (7.8 mm×300 mm, 3 µm), and a mobile phase (0.3 mol/L arginine, 0.2 mol/L sodium chloride, 0.01 mol/L anhydrous calcium chloride, 0.02 mol/L histidine, 0.02% poloxamer 188, pH 7.0)-10% acetonitrile. Elution was carried out isocratically at a flow rate of 0.5 mL/min, a UV detection wavelength of 280 nm, a column temperature of 25°C, and a differential refractive index detector temperature of 30°C. The UV peak area and RI peak area of hFVIII fusion protein, the UV peak area and RI peak area of the purified conjugate solution, and the RI peak area of PEG standard solution were measured and recorded, and standard curves were established using hFVIII fusion protein and PEG standard, respectively. FL1G2-40Y was diluted with a dilution buffer to a concentration of about 0.20 mg/mL, and 100 µL of the dilution was injected into the liquid chromatograph. The average number of PEG modification of the test product was calculated according to the external standard method.

The average number of PEG modification was calculated as follows:
1) Because the PEG moiety in the conjugate had no UV absorption at the wavelength of 280 nm, the UV absorption value of the conjugate (containing the same amount of protein) was the same as that of the fusion protein alone (before modification). The content of the fusion protein part in the conjugate after purification was calculated by the standard curve method. 2) Since the RI absorption value of the fusion protein solution had a linear relationship with the corresponding concentration, the RI value provided by the fusion protein part in the conjugate can be calculated by the standard curve method. 3) Because the RI absorption value of the conjugate was the sum of the RI absorption value of the PEG part and the RI absorption value of the fusion protein part in the conjugate, the RI absorption value of the PEG part in the conjugate can be calculated. 4) Because the RI absorption value of PEG had a linear relationship with the corresponding concentration, the content of the PEG part in the conjugate can be calculated by the standard curve method. 5) The ratio of the molar number of the PEG part in the conjugate to the molar number of the fusion protein part was the average number of PEG modification for one protein in a sample.

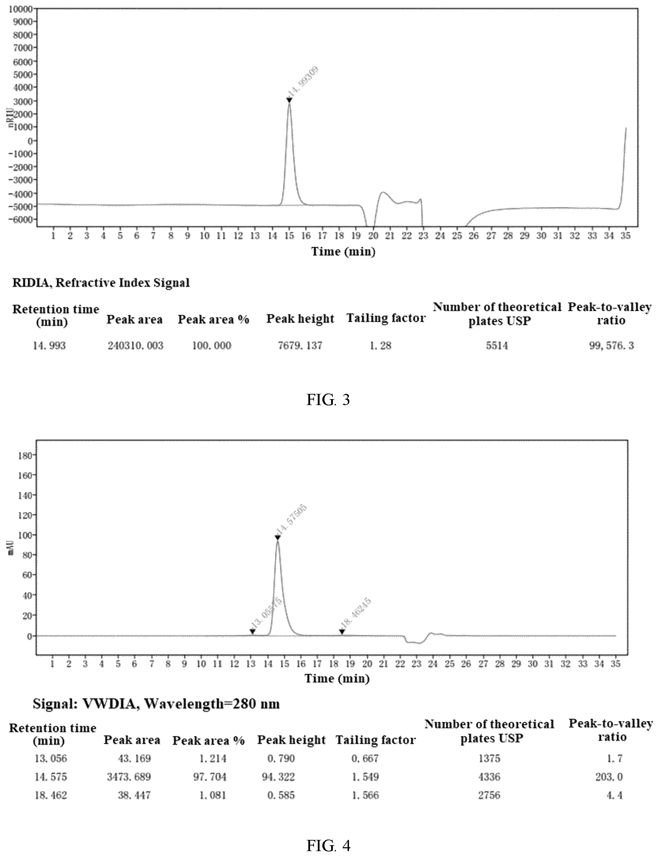

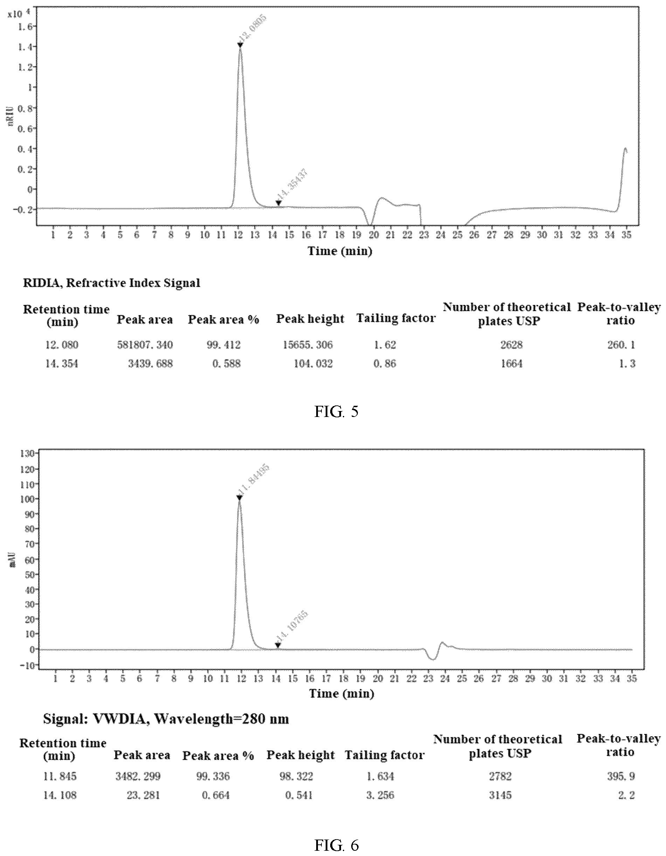

See FIGs. 2-6 for exemplary RI and UV chromatograms. See Tables 2-4 for determination data. The linear equations of the RI peak area and concentration of hFVIII fusion protein and PEG standard were Y=2E+06X-197.15 (R²=0.9998) and Y=1E+06X+1960.1 (R²=0.9998) respectively. The linear equation of the UV peak area and concentration of rhFVIII fusion protein was Y=17590X-13.542 (R²=0.9999). The average values of the RI peak area and UV peak area corresponding to different batches of purified conjugates at 0.20 mg/mL were 579850.509 and 3483.775 respectively. The the average number of PEG modification of the conjugate to be tested was calculated to be about 4.6.

**Table 2. Linear relationship between RI peak area and concentration of hFVIII fusion protein**

| | | | | | |
|---|---|---|---|---|---|
| Concentration of hFVIII fusion protein (mg/ml) | 0.02 | 0.05 | 0.10 | 0.20 | 0.30 |
| RI peak area | 46720.19 | 115063.8 | 235341.1 | 458940.6 | 699441.8 |
| Linear equation and correlation coefficient | Y=2E+06X-197.15, R²=0.9998 | | | | |

**Table 3. Linear relationship between RI peak area and concentration of PEG standard solution**

| | | | | | |
|---|---|---|---|---|---|
| Concentration of PEG (mg/ml) | 0.02 | 0.05 | 0.10 | 0.20 | 0.30 |
| RI peak area | 26869.08 | 61950.3 | 124160 | 240310 | 366588.6 |
| Linear equation and correlation coefficient | Y=1E+06X+1960.1, R²=0.9998 | | | | |

**Table 4. Linear relationship between UV peak area and concentration of hFVIII fusion protein**

| | | | | | |
|---|---|---|---|---|---|
| Concentration of rhFVIII fusion protein (mg/ml) | 0.02 | 0.05 | 0.10 | 0.20 | 0.30 |
| UV peak area | 349.688 | 861.308 | 1750.793 | 3473.689 | 5282.433 |
| Linear equation and correlation coefficient | Y=17590X-13.542, R²=0.9999 | | | | |

The average number of PEG modification of FL1G2-40Y prepared in different batches was determined, and the results were similar. The average number of PEG modification was within 4.2-5.1, see Table 5.

**Table 5 Determination results of the average number of PEG modification in different batches of FL1G2-40Y**

| Batch | Average number of PEG modification |
|---|---|
| P1 | 5.1 |
| P2 | 4.6 |
| P3 | 4.7 |
| P4 | 4.6 |
| P5 | 4.6 |
| P6 | 4.5 |
| P7 | 4.2 |

### Example 3 Direct determination of biological activity of the fusion proteins by one-stage clotting assay

The assay for determination of coagulation factor VIII titer used in the present disclosure is also referred to as one-stage clotting assay. For specific steps, refer to the third part of the Chinese Pharmacopoeia (2020 version). The one-stage clotting assay for FVIII biological activity was based on the ability to correct the FVIII-deficient plasma to prolong the clotting time. The kit Coagulation Factor VIII Deficient Plasma (Cat. No. OTXW17) from Siemens company (German) was used. The assay was performed as: first, the active standard substance WHO FVIII was diluted to 1 IU/ml with 5% FVIII- deficient plasma, and then subjected to a gradient dilution of 10 times, 20 times, 40 times, and 80 times with 5% FVIII-deficient plasma for determination of clotting time. The logarithm of the activity of the known standard substance and the measured clotting time was taken, and then linear fitting was performed to draw the standard curve. The test product was diluted to about 1 IU/ml with 5% FVIII-deficient plasma, and then the above solution was diluted 10 times and 20 times with 5% FVIII-deficient plasma for determination of the clotting time by the same method. The titer of the FVIII sample can be determined according to the standard curve, and the specific activity of the FVIII sample can be calculated accordingly, with a unit of IU/mg. The results show that the specific activities of FL1 G1-40Y and FL1 G2-40Y were 1210 IU/mg and 1300 IU/mg respectively, and the specific activity of FL1G2-40Y was stronger than that of FL1G1-40Y.

### Example 4 Pharmacokinetic assay of PEG-modified hFVIII fusion proteins in HA mice

Eighteen male HA mice were randomly divided into 3 groups, namely FL1G1-40Y group, FL1G2-40Y group and recombinant human coagulation factor VIII for injection (Xyntha) group as a control, 6 mice/group. All groups were given a single intravenous injection at 200 IU/kg. Blood samples (about 0.12 mL) were collected from the non-administration site of the subcutaneous vein of the animal's hind limbs into 1.5 mL sodium citrate blood collection tubes (1:9). The blood was collected at 0 h before administration, 10 min, 1 h, 2 h, 6 h, 12 h, 24 h, 32 h, 48 h, 72 h and 96 h after administration. The blood was centrifuged at 2-8°C (at about 5000 rpm for 5 min) within 30 min after collected. The plasma sample was separated, divided into 2 parts and stored in an ultra-low temperature refrigerator (-70°C~-90°C). The blood samples were collected and centrifuged within 2 hours. The drug concentration in plasma was determined and analyzed using the verified ELISA method. The kinetic parameters of each administration group were calculated using the non-compartmental model method of Phoenix WinNonlin software (Certara L.P., version 8.2). The results of kinetic parameters of each group are shown in Table 6. The results show that at the same dose, the half-lives of the control drug Xyntha, FL1G1-40Y and FL1G2-40Y were 9.46 h, 15.53 h and 20 h, respectively. This shows that after being modified with PEG, the half-life of FL1G2-40Y was increased by 2.11 and 1.29 times compared with Xyntha and FL1G1-40Y respectively, showing a significantly prolonged half-life, which is not reported in the prior art.

**Table 6. PK parameters in HA mice**

| Parameter | Xyntha | FL1G1-40Y | FL1G2-40Y |
|---|---|---|---|
| T_{1/2}(h) | 9.46±0.76 | 15.53±2.99 | 20±3.83 |
| Cₘₐₓ(IU/mL) | 4.87±0.82 | 4.28±0.44 | 4.58±1.29 |
| AUC(h*IU/mL) | 57.78±6.56 | 94.51±8.96 | 119.25±7.48 |
| Vz(mL/kg) | 46.16±7.19 | 47.5±3.43 | 43.65±5.22 |
| CL(mL/h/kg) | 3.37±0.36 | 2.15±0.29 | 1.53±0.14 |
| MRT(h) | 12.21±1.28 | 16.01±4.97 | 22.32±3.12 |

### Example 5 Pharmacokinetic assay of PEG-modified hFVIII fusion protein in cynomolgus monkeys

Twenty-four cynomolgus monkeys were randomly divided into 4 groups (6/group, half male and half female). Groups 1-3 were given a single intravenous injection of 50, 125, 300 IU/kg of FL1G2-40Y, and Group 4 was administered with Xyntha at 125 IU/kg. Blood samples (about 1.8 mL) were collected from the non-administration site of the subcutaneous vein of the animal's hind limbs into 2 mL sodium citrate blood collection tubes (1:9). The blood was collected at 0 h before administration, 10 min, 1 h, 2 h, 6 h, 12 h, 24 h, 32 h, 48 h, 72 h, 96 h, 120 h and 168 h after administration. The blood was centrifuged at 2-8°C (at about 4000 rpm for 5 min) within 30 min after collected. The plasma sample was separated, divided into 2 parts and stored in an ultra-low temperature refrigerator (-70°C~-90°C). The blood samples were collected and centrifuged within 2 hours. The drug concentration in plasma was determined and analyzed using the verified ELISA method. The kinetic parameters of each administration group were calculated using the non-compartmental model method of Phoenix WinNonlin software (Certara L.P., version 8.2). The results of kinetic parameters of each group are shown in Table 7. The results show that at the same dose of 125 IU/kg, the half-lives of the control drug Xyntha and FL1G2-40Y were 7.57 h and 27.8 h, respectively. Compared with Xyntha, the half-life of FL1G2-40Y was increased by 3.67 times, which is not reported in the prior art.

**Table 7. PK parameters in cynomolgus monkeys**

| | Xyntha | FL1G2-40Y | | |
|---|---|---|---|---|
| Dose (IU/kg) | 125 | 50 | 125 | 300 |
| T_{1/2}(h) | 7.57±2.25 | 35.2±10.8 | 27.8±2.25 | 36.8±9.34 |
| Cₘₐₓ(IU/mL) | 1.99±0.26 | 0.99±0.08 | 2.17±0.58 | 6.82±1.14 |
| AUC(h*IU/mL) | 11.6±2.68 | 27.8±5.17 | 61.6±19.1 | 257±71.5 |
| Vz(mL/kg) | 28.8±3.22 | 74.5±10.8 | 30.9±5.06 | 9.29±1.23 |
| CL(mL/h/kg) | 2.94±0.90 | 1.72±0.30 | 0.83±0.20 | 0.199±0.06 |
| MRT(h) | 4.17±0.97 | 34.6±9.07 | 32.9±4.59 | 40.8±5.60 |

### Example 6 Pharmacokinetic assay of PEG-modified hFVIII fusion protein in patients with severe hemophilia A

Inclusion criteria: eligible subjects must meet the following requirements: 1) 12 years old ≤ age < 60 years old, male; 2) patients with clinically diagnosed severe hemophilia A (coagulation factor VIII <1%), having previous medical records confirming receiving of treatment with factor VIII (EDs ≥ 150); 4) non-acute bleeding state; 5) negative for previous coagulation factor VIII inhibitor (<0.6 BU), no family history of inhibitors; 6) platelet counts > 100,000 cells/ µL; 7) prothrombin time is normal or INR<1.3; 8) thrombin time (TT) is normal; 9) previous vWF antigen test results are normal; 10) negative for lupus anticoagulant.

Clinical design: The experiment was set up with 2 dose groups, namely 25 IU/kg group and 50 IU/kg group respectively, with no less than 6 effective cases in each group. The subjects were first administered with the control drug Advate (recombinant human coagulation factor VIII for injection, ADVATE) in a single dose, and then administered with the test drug in a single dose. The subjects in each group were admitted to the clinical trial center on the day before the administration (Day -1), fasting on the morning of the administration day, and administered intravenously.

The concentration of the drug in plasma was determined and analyzed using the verified ELISA method, and the kinetic parameters of each administration group were calculated using the non-compartmental model method (NCA) of Phoenix WinNonlin software (Certara L.P., version 8.2). The results of pharmacokinetic parameters in each group are shown in Table 8.

**Table 8. PK parameters in patients with severe hemophilia A**

| | Advate | FL1G2-40Y | Advate | FL1G2-40Y |
|---|---|---|---|---|
| Dose (IU/kg) | 25 | 25 | 50 | 50 |
| N | 6 | 6 | 7 | 7 |
| T_{1/2}(h) | 14.18±4.53 | 31.91±9.52 | 14.68±5.50 | 30.87±2.18 |
| Cₘₐₓ(IU/dL) | 70.87±11.51 | 76.57±20.32 | 137.89±27.67 | 134.66±28.33 |
| AUC(h*IU/dL) | 874.17±187.70 | 2889.87±556.84 | 1834.73±418.24 | 5772.29±1916.86 |
| Vz(dL/kg) | 0.60±0.24 | 0.41±0.14 | 0.57±0.15 | 0.41±0.11 |
| Vss(dL/kg) | 0.45±0.11 | 0.34±0.09 | 0.44±0.09 | 0.38±0.10 |
| CL(dL/h/kg) | 0.029±0.0054 | 0.009±0.002 | 0.028±0.0066 | 0.009±0.0027 |
| MRT(h) | 13.61±1.81 | 35.63±3.26 | 14.69±2.14 | 40.07±4.83 |

The results show that the half-lives of Advate at 25 IU/kg and 50 IU/kg were 14.18 h and 14.68 h respectively; while the half-lives of FL1G2-40Y at 25 IU/kg and 50 IU/kg were 31.91 h and 30.87 h respectively, which were increased by 2.25 times (25 IU/kg) and 2.10 times (50 IU/kg) respectively compared with those of Advate. At a dose of 50 IU/kg, the marketed drug Xyntha has a half-life of 13.76 h in patients over 12-year old, which is comparable to that of Advate at the same dose. It can be reasonably speculated that the *in vivo* half-life of FL1G2-40Y is also significantly longer than that of Xyntha, showing a significantly prolonged half-life.

The above descriptions are only preferred embodiments of the present invention, and are not intended to limit the present invention. Any modifications, equivalent replacements, improvements and the like made within the spirit and principles of the present invention shall be included within the protection scope of the present invention.

## Claims

1. A conjugate of a coagulation factor VIII-Fc fusion protein and polyethylene glycol (PEG), wherein the coagulation factor VIII-Fc fusion protein comprises an active moiety of the coagulation factor VIII (FVIII) and an Fc fragment, the active moiety of the coagulation factor VIII (FVIII) is directly connected to the Fc fragment or indirectly linked to the Fc fragment through a linker to form the fusion protein, the conjugate is modified with PEG at an average PEG number of 3-8, 3-7, 3-6, 4-8, 4-7, 4-6 or 4.2-5.1, preferably 4.2-5.1, and the average PEG number of modification is the molar ratio of the PEG to the fusion protein.

2. The conjugate according to claim 1, wherein, a modifier for PEG modification has a structure represented by formula (2): 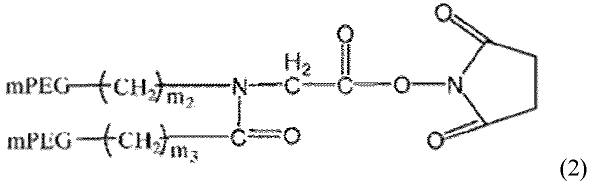
wherein, 0≤m2≤6, preferably, m2 is 2; 0≤m3≤6, preferably, m3 is 1; mPEG-represents a methoxy mono-capped polyethylene glycol group; and
preferably, the PEG has a molecular weight of 30 kD-50 kD, preferably 40 kD.

3. The conjugate according to claim 1 or 2, wherein the active moiety of the coagulation factor VIII is full-length or truncated human coagulation factor VIII, preferably B-domain truncated human coagulation factor VIII, more preferably a polypeptide comprising an amino acid sequence set forth in SEQ ID NO: 1 or SEQ ID NO: 2, or a polypeptide having at least 90%, 95% or higher identity to the amino acid sequence set forth in SEQ ID NO: 1 or SEQ ID NO: 2 and still having FVIII activity.

4. The conjugate according to any one of claims 1 to 3, wherein the Fc fragment is an Fc fragment derived from IgG, preferably IgG1, IgG2 or IgG4, more preferably IgG2.

5. The conjugate according to claim 4, wherein the Fc fragment comprises an amino acid sequence selected from the group consisting of:
(i) the amino acid sequence set forth in SEQ ID NO: 3;
(ii) the amino acid sequence set forth in SEQ ID NO: 4; and
(iii) the amino acid sequence set forth in SEQ ID NO: 5.

6. The conjugate according to any one of claims 1 to 5, wherein the linker comprises a flexible unit and a rigid unit.

7. The conjugate according to claim 6, wherein the flexible unit is represented by a general formula of (GS)ₐ(GGS)_{b}(GGGS)_{c}(GGGGS)_{d}, wherein a, b, c and d are integers greater than or equal to 0, and a+b+c+d≥1,
preferably, the flexible unit comprises an amino acid sequence selected from the group consisting of:
(i) GSGGGSGGGGSGGGGS (SEQ ID NO: 6),
(ii) GSGGGGSGGGGSGGGGSGGGGSGGGGS (SEQ ID NO: 7),
(iii) GGGGSGGGGSGGGGSGGGGS (SEQ ID NO: 8),
(iv) GSGGGGSGGGGSGGGGSGGGGSGGGGSGGGGSGGGGS (SEQ ID NO: 9), and
(v) GGGSGGGSGGGSGGGSGGGS (SEQ ID NO: 10).

8. The conjugate according to claim 6, wherein the rigid unit is a carboxy terminal peptide of human chorionic gonadotropin β subunit,
preferably, the rigid unit comprises an amino acid sequence selected from the group consisting of:
(i) PRFQDSSSSKAPPPPSLPSPSRLPGPSDTPILPQ (SEQ ID NO: 11),
(ii) SSSSKAPPPSLPSPSRLPGPSDTPILPQ (SEQ ID NO: 12),
(iii) SSSSKAPPPS (SEQ ID NO: 13),
(iv) SRLPGPSDTPILPQ (SEQ ID NO: 14), and
(v)GSGGGGSGGGGSGGGGSGGGGSGGGSSSSSKAPPPPSLPSPSRLPGPSDTPILPQ (SEQ ID NO: 15).

9. The conjugate according to any one of claims 1 to 8, wherein the coagulation factor VIII-Fc fusion protein sequentially comprises, from the nitrogen terminal to the carbon terminal, the B-domain truncated human coagulation factor VIII, the flexible unit, the rigid unit and the Fc fragment; wherein,
the B-domain truncated human coagulation factor VIII comprises an amino acid sequence set forth in SEQ ID NO: 2, the flexible unit comprises an amino acid sequence set forth in SEQ ID NO: 7, the rigid unit comprises an amino acid sequence set forth in SEQ ID NO: 12, the Fc fragment comprises an amino acid sequence set forth in SEQ ID NO: 4;
the B-domain truncated human coagulation factor VIII comprises an amino acid sequence set forth in SEQ ID NO: 2, the flexible unit comprises an amino acid sequence set forth in SEQ ID NO: 6, the rigid unit comprises an amino acid sequence set forth in SEQ ID NO: 11, the Fc fragment comprises an amino acid sequence set forth in SEQ ID NO: 4;
the B-domain truncated human coagulation factor VIII comprises an amino acid sequence set forth in SEQ ID NO: 2, the flexible unit comprises an amino acid sequence set forth in SEQ ID NO: 7, the rigid unit comprises an amino acid sequence set forth in SEQ ID NO: 12, the Fc fragment comprises an amino acid sequence set forth in SEQ ID NO: 5;
or
the B-domain truncated human coagulation factor VIII comprises an amino acid sequence set forth in SEQ ID NO: 2, the flexible unit comprises an amino acid sequence set forth in SEQ ID NO: 6, the rigid unit comprises an amino acid sequence set forth in SEQ ID NO: 11, the Fc fragment comprises an amino acid sequence set forth in SEQ ID NO: 5.

10. The conjugate according to claim 9, wherein:
the B-domain truncated human coagulation factor VIII comprises an amino acid sequence set forth in SEQ ID NO: 2, the flexible unit comprises an amino acid sequence set forth in SEQ ID NO: 7, the rigid unit comprises an amino acid sequence set forth in SEQ ID NO: 12, the Fc fragment comprises an amino acid sequence set forth in SEQ ID NO: 4;
the PEG molecule is linked to the primary amine (-NH₂) group on the lysine residue in the coagulation factor VIII-Fc fusion protein through an active group succinimidyl carboxymethyl ester (SCM);
the modifier for PEG modification has a structure represented by formula (2): 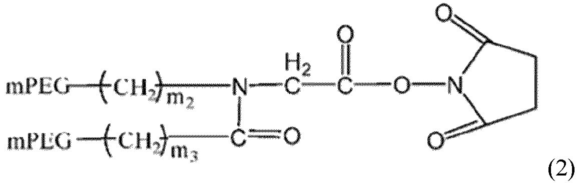
wherein, m2 is 2; m3 is 1; the molecular weight of the PEG is 40 kD.

11. A pharmaceutical composition comprising the conjugate according to any one of claims 1 to 10, and a pharmaceutically acceptable carrier.

12. Use of the conjugate according to any one of claims 1 to 10 in the manufacture of a medicament for preventing and/or treating hemorrhagic diseases.

13. The use according to claim 12, wherein the hemorrhagic disease is selected from the group consisting of hemorrhagic diseases in patients with congenital or acquired deficiency of FVIII, and spontaneous or surgical bleeding in patients with hemophilia A.

14. A method for preventing and/or treating hemorrhagic diseases, comprising administering the conjugate according to any one of claims 1 to 10 to a subject in need thereof, preferably, the hemorrhagic disease is selected from the group consisting of hemorrhagic diseases in patients with congenital or acquired deficiency of FVIII, and spontaneous or surgical bleeding in patients with hemophilia A.

15. A method for preparing the conjugate according to any one of claims 1 to 10, comprising:
1) preparing the coagulation factor VIII-Fc fusion protein;
2) reacting the fusion protein obtained in step 1) with PEG, wherein the PEG and the fusion protein are in a molar ratio of (50-120): 1, preferably 100: 1, and the PEG has a molecular weight of 30-50 kD, preferably, the PEG is a branched PEG of 40 kD; and
3) purifying the conjugate obtained in step 2).
